# EUROPEAN PATENT APPLICATION

(11) **EP 4 691 488 A1**
(43) Date of publication of application: **11.02.2026**
(21) Application number: 24780840.5
(22) Date of filing: 29.03.2024
(51) Int. Cl.: A61K 39/395, A61P 11/00, G01N 33/53, C07K 16/24

(54) **THERAPEUTIC DRUG FOR INTERSTITIAL LUNG DISEASE**

(30) Priority: 29.03.2023 JP 2023054230
(71) Applicant: NAGASAKI UNIVERSITY, Nagasaki 852-8521 (JP); Mabprotein Co.,Ltd., Izumo-shi, Shimane, 693-8501 (JP)
(72) Inventor: KAWAKAMI, Atsushi, Nagasaki-shi, Nagasaki 852-8521 (JP); KOGA, Tomohiro, Nagasaki-shi, Nagasaki 852-8521 (JP); SHIMIZU, Toshimasa, Nagasaki-shi, Nagasaki 852-8521 (JP); KURUSHIMA, Shota, Nagasaki-shi, Nagasaki 852-8521 (JP); SAKAMOTO, Noriho, Nagasaki-shi, Nagasaki 852-8521 (JP); URANO, Takeshi, Izumo-shi, Shimane 693-8501 (JP); NARIAI, Yuko, Izumo-shi, Shimane 693-8501 (JP); KAMINO, Hiroki, Izumo-shi, Shimane 693-8501 (JP)
(74) Representative: Schön, Christoph
(86) International application number: PCT/JP2024/013111
(87) International publication number: WO 2024/204745

(57) **Abstract**

Although it has been implicated that interstitial lung diseases are associated with IL-18, there has been no effective therapeutic drug. It was demonstrated that administration of an anti-active IL-18 antibody that recognizes a neo-epitope of active IL-18 to a mouse model with an interstitial lung disease induced by bleomycin suppressed pulmonary fibrosis both biochemically and histopathologically. IL-18BP and antibody that recognize regions other than the neo-epitope of the IL-18 have not been observed to exhibit therapeutic effects. Accordingly, the anti-active IL-18 antibody can be an effective therapeutic drug for interstitial lung diseases for which no effective therapeutic drug has previously been available, particularly for progressive interstitial lung diseases.

## Description

### Technical Field

The present invention relates to a therapeutic drug for an interstitial lung disease (interstitial pneumonia).

### Background Art

Interstitial lung diseases (ILDs) are inflammatory pathological conditions that occur in the interstitium of the lungs, in which fibrosis develops between the pulmonary alveoli and capillary vessels, resulting in impaired gas exchange. Examples of the cause of the interstitial lung diseases include long-term exposure to inhaled substances such as tobacco smoke, mold, dust, and asbestos; autoimmune diseases such as rheumatoid arthritis, polymyositis, and dermatomyositis; adverse effects caused by a drug such as anticancer agents or antibacterial agents; and infectious diseases such as COVID-19 and Mycoplasma infectious diseases. In some cases, the cause of the interstitial lung disease can be identified, and in other cases, including idiopathic interstitial pneumonias, the cause cannot be identified. Among diseases of which the cause cannot be identified, a representative disease is idiopathic pulmonary fibrosis.

It is reported that the number of patients affected by an interstitial lung disease who visit a medical institution is 10 to 20 per 100,000 people. However, since the interstitial lung diseases are asymptomatic at the early stage, it is inferred that the number of potentially affected individuals is ten or more times higher. When the cause of an interstitial lung disease is known, as in the case of drug-induced or infection-related ILD, it is expected that the disease is cured by an adequate treatment targeting the underlying cause. However, for many progressive interstitial lung diseases, there is currently no definitive treatment.

It is known that interleukin-18 (IL-18) increases in various inflammatory diseases, and it has been implicated that the IL-18 is involved in interstitial lung diseases. The IL-18 is an inflammatory cytokine that is produced as a precursor and is converted to its active form in an inflammasome through caspase-dependent pathway, and exerts a physiological activity. Pulmonary alveolar epithelial cells strongly express the IL-18 and IL-18Rα (Non Patent Literature 1). In mice with pulmonary fibrosis induced by bleomycin, which serve as a model of inflammatory pulmonary fibrosis, the efficacy of an IL-18 binding protein (IL-18BP) that neutralizes IL-18, has been demonstrated (Non Patent Literature 2). Therefore, the IL-18 is considered to be a highly promising novel therapeutic target that can achieve both suppression of the fibrosis and regulation of the inflammation. However, the effect of the IL-18BP to suppress the fibrosis has only been observed with daily administration, and in view of its short half-life, clinical application of the IL-18BP is considered impractical.

As a drug that inhibits IL-18, the use of an anti-IL-18 antibody has been considered. Since it has been implicated that the IL-18 is involved in many diseases, several companies are already developing anti-IL-18 antibody pharmaceuticals. For such pharmaceuticals, phase II clinical trials have been conducted for sarcoidosis (NCT04064242), atopic dermatitis (NCT04836858), transplant rejection (NCT02723786), type 2 diabetes (NCT01648153), Behcet's disease (NCT03522662), and Crohn's disease (NCT03681067); and phase I clinical trials have been conducted for inflammatory bowel disease (NCT01035645), adult-onset Still's disease (NCT04752371), and atopic dermatitis (NCT04975438). However, none has advanced to a phase III clinical trial, and no clinical trials have yet been conducted for an interstitial lung disease. Furthermore, it has become clear that IL-18 inhibitors are not effective in all diseases in which IL-18 elevation is observed. As mentioned above, although the IL-18BP has been shown to be effective against an interstitial lung disease, a therapeutic method based on inhibition of the IL-18 has not yet been established.

### Citation List

### Patent Literature

Patent Literature 1: International Publication No. WO 2014/080866
Patent Literature 2: International Publication No. WO 2020/116423
Patent Literature 3: International Publication No. WO 2019/194217
Patent Literature 4: Japanese Translation of PCT International Application Publication No. 2014-508511
Patent Literature 5: International Publication No. WO 2023/286694
Patent Literature 6: International Publication No. WO 2004/092219

### Non Patent Literature

Non Patent Literature 1: Kitasato Y. et al., Am J Respir Cell Mol Biol 2004, Vol. 31, pp. 619-625
Non Patent Literature 2: Zang L. M. et al., Biochem Biophys Res Commun 2019, Vol. 508, pp. 660-666
Non-Patent Literature 3: Hezareh, M. et al., J. Virol. 2001, Vol. 75, pp. 12161-12168
Non Patent Literature 4: Oganesyan, V. et al., Acta Cryst., 2008, D64, pp. 700-704
Non-Patent Literature 5: Tao, M. and Morrison, S. L., J. Immunol., 1989, Vol. 143, pp. 2595-2601
Non-Patent Literature 6: Shields, R. L. et al., J. Biol. Chem., 2001, Vol. 276, pp. 6591-6604
Non-Patent Literature 7: Arimori, T. et al., Structure, 2017, Vol. 25, pp. 1611-1622
Non-Patent Literature 8: Gono, T. et al., Rheumatology 2010, Vol. 49, pp. 1878-1881
Non-Patent Literature 9: Bohan, A. and Peter, J.B., N Engl J Med, 1975, Vol. 292, pp. 344-347
Non Patent Literature 10: Lundberg, I. E. et al., Ann Rheum Dis 2017, Vol. 76, pp. 1955-1964
Non Patent Literature 11: Sontheimer, R. D., J Am Acad Dermatol 2002, Vol. 46, pp. 626-636

### Summary of Invention

### Technical Problem

Since it has been suspected that the IL-18 and the IL-18Rα are involved in the pathological condition of the interstitial lung diseases, the possibility of therapy using an anti-IL-18 antibody has been mentioned so far (Patent Literatures 1 to 4). However, in any of these literatures, such a possibility is merely suggested, and none demonstrates in its Examples the effect of an anti-IL-18 antibody on an interstitial lung disease. Further, no clinical trial or the like has ever been conducted in which a therapy using an anti-IL-18 antibody was attempted for an interstitial lung disease. It is an object of the present invention to provide a therapeutic drug that is effective for any pathological condition of interstitial lung disease, such as chronic interstitial lung disease or progressive interstitial lung disease for which no effective drug is available.

### Solution to Problem

The present invention relates to a pharmaceutical composition, a method of predicting a therapeutic effect, and a treating method for interstitial lung disease as follows.
(1) A pharmaceutical composition for treating an interstitial lung disease, including as an active ingredient an anti-active IL-18 neutralizing antibody, or a fragment thereof selected from Fab, Fab', F(ab')₂, single chain antibody (scFv), disulfide stabilized V region fragment (dsFv), antibody with a reduced molecular weight (Fv-clasp), or a peptide including a CDR.
(2) The pharmaceutical composition for treating an interstitial lung disease according to (1), wherein the interstitial lung disease is an interstitial lung disease associated with polymyositis and/or dermatomyositis.
(3) The pharmaceutical composition for treating an interstitial lung disease according to (1) or (2), wherein the anti-active IL-18 neutralizing antibody includes a heavy chain variable domain or an antigen binding site that binds to an N-terminal region of active IL-18, including a CDR1H region consisting of the amino acid sequence GFSLSSSGMG (SEQ ID NO: 1), a CDR2H region consisting of the amino acid sequence IWWDDDK (SEQ ID NO: 2), and a CDR3H region consisting of the amino acid sequence TRTRTYSNFGGGMAY (SEQ ID NO: 3), and a light chain variable domain of the anti-active IL-18 neutralizing antibody is a peptide including a CDR1L region consisting of the amino acid sequence QSIAHSNGYTY (SEQ ID NO: 4), a CDR2L region consisting of the amino acid sequence KVS, and a CDR3L region consisting of the amino acid sequence VQGSHVPLT (SEQ ID NO: 5); or wherein the heavy chain variable domain of the anti-active IL-18 neutralizing antibody includes a CDR1H region consisting of the amino acid sequence GFSLTSYG (SEQ ID NO: 6), a CDR2H region consisting of the amino acid sequence IWAGGST (SEQ ID NO: 7), and a CDR3H region consisting of the amino acid sequence ARESSYDAMDY (SEQ ID NO: 8), and the light chain variable domain of the anti-active IL-18 neutralizing antibody is a peptide including a CDR1L region consisting of the amino acid sequence ENVVTY (SEQ ID NO: 9), a CDR2L region consisting of the amino acid sequence GAS, and a CDR3L region consisting of the amino acid sequence GQGYSYPYT (SEQ ID NO: 10).
(4) The pharmaceutical composition for treating an interstitial lung disease according to (1) or (2), wherein the amino acid sequence of the H chain variable region of the anti-active IL-18 neutralizing antibody or the antigen binding site binding to the N-terminal region of the active IL-18 is SEQ ID NO: 11, and the amino acid sequence of the L chain variable region is SEQ ID NO: 12; or wherein the amino acid sequence of the H chain variable region is SEQ ID NO: 13 and the amino acid sequence of the L chain variable region is SEQ ID NO: 14.
(5) A therapeutic effect prediction method for a patient with an interstitial lung disease as a subject to determine in advance whether the pharmaceutical composition for treating the interstitial lung disease according to any one of (1) to (4), the method including measuring an expression level of IL-18 in the subject.
(6) A method of treating an interstitial lung disease including administering the pharmaceutical composition for treating the interstitial lung disease according to any one of (1) to (4) to a patient with the interstitial lung disease.
(7) A method of treating an interstitial lung disease, including confirming in advance therapeutic efficacy of a treatment with an anti-active IL-18 neutralizing antibody by the method according to (5), and performing the treatment by the method according to (6).

### Brief Description of Drawings

[Figure 1] Figure 1 is a diagram showing results of measuring concentrations of IL-18 in the serum of patients with dermatomyositis complicated by an interstitial lung disease.
[Figure 2A] Figure 2A is a diagram showing results of measuring concentrations of active IL-18 in the serum of patients with dermatomyositis complicated by an interstitial lung disease (PM/DM-ILD).
[Figure 2B] Figure 2B is a diagram showing results of measuring concentrations of the active IL-18 in the serum of patients with PM/DM complicated or not complicated by an ILD.
[Figure 2C] Figure 2C is a diagram showing results of measuring concentrations of the active IL-18 in the serum of patients with acute, subacute, and chronic PM/DM-ILD.
[Figure 3A] Figure 3A is a diagram showing results of measuring concentrations of the active IL-18 in bronchoalveolar lavage fluid of patients with PM/DM-ILD.
[Figure 3B] Figure 3B is a diagram showing results of measuring concentrations of the active IL-18 in the bronchoalveolar lavage fluid of patients with acute, subacute, and chronic PM/DM-ILD.
[Figure 3C] Figure 3C is a diagram showing the correlation between the concentrations of the active IL-18 in the serum and the bronchoalveolar lavage fluid from the same patient.
[Figure 4A] Figure 4A is a diagram showing the homology between the amino acid sequences of human and mouse IL-18.
[Figure 4B] Figure 4B is a diagram showing results of examining an epitope of the 5-4.1 antibody that recognizes the mouse active IL-18 by the alanine scanning method.
[Figure 5] Figure 5 is a schematic diagram of the experimental schedule using pulmonary fibrosis mouse models.
[Figure 6] Figure 6 is a diagram showing the effects of drugs as determined by the amount of hydroxyproline, a biochemical marker of the pulmonary fibrosis, in the pulmonary fibrosis mouse models.
[Figure 7] Figure 7 is a diagram showing histopathological images of lungs from each experimental group.
[Figure 8] Figure 8 is a diagram showing a result of a comparison between the effects of the anti-active IL-18 antibody and the anti-IL-18 antibody in the pulmonary fibrosis mouse model.

### Description of Embodiments

Although there are numerous diseases in which the IL-18 increases upon the disease onset, and it is suspected that the IL-18 is involved in many diseases, there are few diseases in which a clear causal involvement of IL-18 has been demonstrated. As an example in which a therapeutic effect of an anti-IL-18 antibody is demonstrated, there is only a case in which the therapeutic effect was analyzed by the inventors' group in an inflammatory bowel disease model using an antibody that recognizes only the active form of IL-18 (anti-active IL-18 antibody) (Patent Literature 5). In the present study, the inventors have confirmed that the anti-active IL-18 antibody is effective in a model of interstitial lung disease, and completed the present invention.

The anti-IL-18 antibody of the present invention is an antibody that recognizes only the active form of IL-18; that is, it recognizes a neo-epitope newly generated when the precursor pro-IL-18 is cleaved by caspase-1 or caspase-4. In the present specification, an antibody that recognizes only the active form of IL-18 is referred to as an antibody recognizing a neo-epitope, or as an anti-active IL-18 antibody.

The term "epitope" refers to a part of an antigen recognized by an antibody, and in the present specification, it means the site on an antigen to which the domain containing the antibody variable region disclosed herein binds. For example, when a polypeptide such as the IL-18 serves as an antigen, an antibody may recognize a linear amino acid sequence or a three-dimensional conformational structure. Accordingly, an epitope can be defined based on the amino acid sequence or the structure of the antigen. The neo-epitope of the IL-18 is a sequence present only in the active IL-18 that has been cleaved by caspase-1 or caspase-4, and an antibody recognizing this sequence can be regarded as an antibody that recognizes only the active form of the IL-18. It should be noted that in the present specification the terms "antibody that recognizes only the active form of IL-18" and "antibody that recognizes a neo-epitope" are used interchangeably.

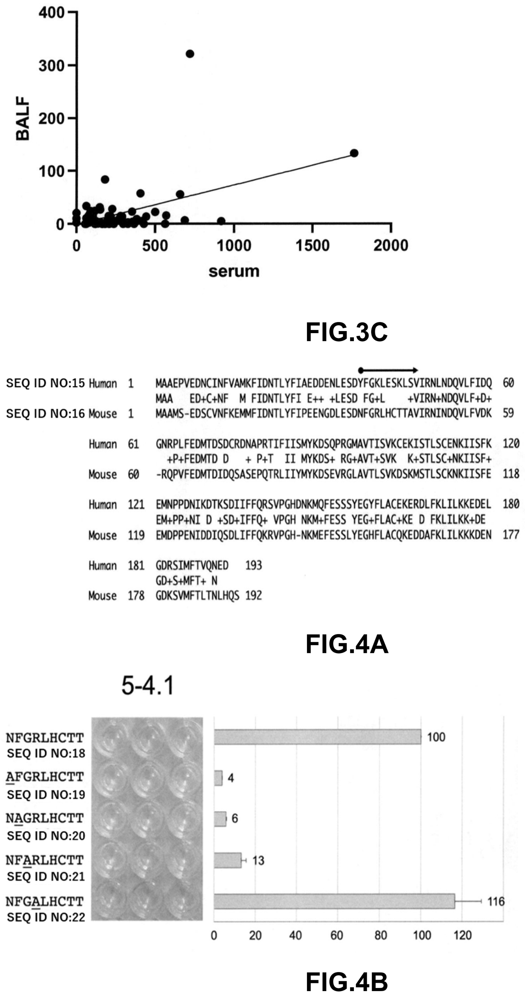

Further, in the Examples of the present invention, since the N-terminal sequence of the active IL-18 differs between humans and mice (see Figure 4A), an antibody that recognizes mouse active IL-18 was used. However, it goes without saying that for application in humans, an antibody that recognizes only human active IL-18 should be used.

As antibodies that recognize active human IL-18, the present inventors have developed antibodies designated 9-10.2 and 8-4.1 (Patent Literature 2). Specifically, such antibodies include an antibody which includes a heavy chain variable domain containing a CDRH1 region consisting of the amino acid sequence GFSLSSSGMG (SEQ ID NO: 1), a CDRH2 region consisting of the amino acid sequence IWWDDDK (SEQ ID NO: 2), and a CDRH3 region consisting of the amino acid sequence TRTRTYSNFGGGMAY (SEQ ID NO: 3), and a light chain variable domain containing a CDRL1 region consisting of the amino acid sequence QSIAHSNGYTY (SEQ ID NO: 4), a CDRL2 region consisting of the amino acid sequence KVS, and a CDRL3 region consisting of the amino acid sequence VQGSHVPLT (SEQ ID NO: 5) (the 9-10.2 antibody) ; and an antibody which includes a heavy chain variable domain containing a CDRH1 region consisting of the amino acid sequence GFSLTSYG (SEQ ID NO: 6), a CDRH2 region consisting of the amino acid sequence IWAGGST (SEQ ID NO: 7), and a CDRH3 region consisting of the amino acid sequence ARESSYDAMDY (SEQ ID NO: 8), and a light chain variable domain containing a CDRL1 region consisting of the amino acid sequence ENVVTY (SEQ ID NO: 9), a CDRL2 region consisting of the amino acid sequence GAS, and a CDRL3 region consisting of the amino acid sequence GQGYSYPYT (SEQ ID NO: 10) (the 8-4.1 antibody). Further, the antibody in which the amino acid sequence of the H chain variable region is SEQ ID NO: 11 and the amino acid sequence of the L chain variable region is SEQ ID NO: 12 (the 9-10.2 antibody), the antibody in which the H chain variable region is SEQ ID NO: 13 and the amino acid sequence of the L chain variable region is SEQ ID NO: 14 (the 8-4.1 antibody), or an antibody having 80% or more homology, preferably 90% or more homology, more preferably 95% or more homology, and even more preferably 98% or more homology, to these antibodies, can be used.

Further, although the 9-10.2 antibody and the 8-4.1 antibody that recognize the human active IL-18 are mouse monoclonal antibodies, for clinical application in humans, the present invention also encompasses humanized antibodies obtained from these monoclonal antibodies by genetic recombination techniques such as a human chimeric antibody or a humanized CDR-grafted antibody, and a human antibody produced using a genetically engineered mouse. When administered to a human, the humanized antibody and the human antibody exhibit fewer side effects and longer-lasting therapeutic efficacy compared with antibodies derived from non-human animals. Furthermore, by introducing mutations into the Fc region of human IgG1 so as to decrease the binding ability to C1q and Fc receptors, it is possible to prevent the antibody from being internalized by immune cells such as macrophages and increase the neutralizing activity of the antibody. Examples of such mutations include K322A, L234A, L235A (Non Patent Literature 3); the triple mutation L234F/L235E/P331S (Non Patent Literature 4); and N297Q (Non Patent Literatures 5 and 6). Based on these findings, for example, LALA mutation (L234A and L235A) may be introduced to an antibody so that uptake thereof by immune cells such as macrophages is suppressed (Patent Literature 6). It should be noted that the present inventors have already produced a humanized antibody that recognizes the neo-epitope and confirmed that it exhibits sufficient neutralizing activity.

In the present specification, the term "antibody" encompasses functional fragments that exhibit substantially the same antigen specificity as the original antibody, i.e., that recognize the same epitope. The functional antibody fragments of the antibody include Fab, Fab', F(ab')₂, single chain antibody (scFv), disulfide stabilized V region fragment (dsFv), antibody with a reduced molecular weight (Fv-clasp; Non Patent Literature 7), and peptides including a CDR, among others.

As noted above, interstitial lung disease can be caused by various factors. Thus, it is considered that the extent of the involvement of the IL-18 in disease onset may vary. The pharmaceutical composition of the present invention is an antibody that acts on and neutralizes the active IL-18. Accordingly, it is expected to be particularly effective in patients with an interstitial lung disease in which the active IL-18 is detected. In order to determine whether a therapeutic effect is achieved by the anti-active IL-18 antibody in a patient, a concentration of the IL-18, particularly a concentration of the active IL-18, in a blood sample from the patient, may be measured. The IL-18 may be measured either by using an existing kit or by a method commonly employed in the field, such as ELISA, using an anti-active IL-18 antibody that recognizes the active IL-18 (that is, the neo-epitope), such as the 9-10.2 antibody or the 8-4.1 antibody. If the concentration of the IL-18 in the blood is higher than that in a healthy individual, it can be determined that the anti-active IL-18 antibody pharmaceutical is effective.

As a method of administering the pharmaceutical composition of the present invention to a patient, parenteral administration, that is, subcutaneous administration, intramuscular administration, intravenous administration, intraperitoneal administration, or intranasal administration is useful. Preferred embodiments include administration by subcutaneous or intramuscular injection. Other preferred embodiments include administration by intravenous drip infusion or intravenous injection. Further, when used as an injectable liquid preparation, it is preferably prepared as an injectable liquid preparation containing 0.1 to 250 mg/mL of the antibody or the functional fragment of the antibody as the active ingredient of the present invention. Moreover, the pharmaceutical composition may further contain a pharmaceutically acceptable carrier. Specific examples of the pharmaceutically acceptable carrier include all physiologically acceptable solvents, dispersants, antibacterial agents or antifungal agents, isotonic agents, absorption delaying agents, and the like. Specific examples of the pharmaceutically acceptable carrier include water, physiological saline, phosphate buffered saline, dextrose, glycerin, ethanol, and the like. At least one or more of these carriers may be contained. Further, the pharmaceutically acceptable carrier may auxiliary contain small amounts of substances that enhance the storage life or efficacy of the antibody or the antibody fragment, such as a wetting agent, an emulsifying agent, a preservative agent, and a buffer agent. As for dosing intervals, treatment should preferably be continued for an appropriate period while monitoring therapeutic effects. Since it is an antibody preparation, repeated administration every 2-4 weeks is preferred.

Below, clinical cases of polymyositis (PM) and dermatomyositis (DM) will be described. However, as clearly demonstrated by the verification using an animal model with an interstitial lung disease induced by bleomycin, it is needless to say that the therapeutic drug of the present invention is effective for interstitial lung diseases caused by various factors. The polymyositis and the dermatomyositis are one of connective tissue diseases, which are autoimmune diseases, and are characterized by inflammation of the muscles, causing symptoms such as muscle weakness, general fatigue, and easy fatigability. They are classified into polymyositis without skin symptoms and dermatomyositis with skin symptoms. Although they are likely distinct diseases, the distinction is not clear, and the causes remain unknown. Since the interstitial lung disease accounts for approximately 50% of deaths among patients with polymyositis or dermatomyositis, the interstitial lung disease is a representative complication of these diseases. Currently, the polymyositis and the dermatomyositis are treated with corticosteroids, immunosuppressing agents, and nintedanib, an antifibrotic drug. However, approximately 20 to 30% of patients exhibit a progressive phenotype, and at least for this patient subset, no effective existing therapy is available.

The present invention will now be described in detail with reference to experimental data.

### [Example 1] Concentration of IL-18 in Serum of Patients with Polymyositis/Dermatomyositis

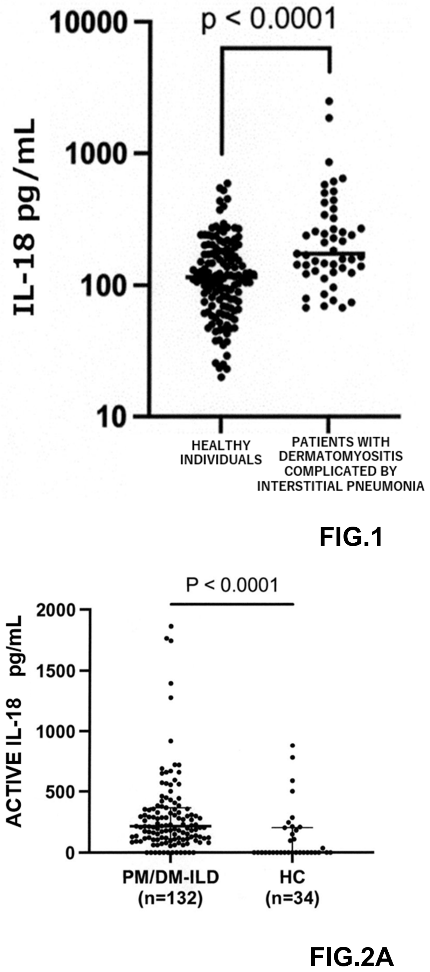

It has been reported that serum IL-18 levels are elevated in patients with polymyositis/dermatomyositis complicated by an interstitial lung disease (hereinafter, may also be referred to sometimes referred to as "PM/DM-ILD") (Non Patent Literature 8). Accordingly, serum IL-18 concentrations were measured in 49 PM/DM-ILD patients and 133 healthy subjects as controls (Figure 1).

The PM/DM-ILD patients consisted of 49 individuals diagnosed with polymyositis or dermatomyositis between July 2009 and February 2018 at Nagasaki University Hospital and its affiliated institutions. Diagnosis of PM/DM was based on the criteria of Bohan and Peter (Non Patent Literature 9) and those of EULAR/ACR (the criteria of the European Alliance of Associations for Rheumatology/American College of Rheumatology; Non Patent Literature 10). Diagnosis of clinically amyopathic dermatomyositis (CADM) was based on the criteria of Sontheimer and Gerami et al. (Non Patent Literature 11) and the EULAR/ACR criteria (Non Patent Literature 10). The presence or absence of the interstitial lung disease was evaluated using results from high-resolution computed tomography (HRCT) of the chest.

Blood samples were collected from the PM/DM-ILD patients and the healthy individuals and centrifuged at 3000 × g for five minutes. The supernatants were collected and stored at -20°C until analysis. The concentrations of the IL-18 in the serum were measured using the Bio-Plex Pro (trademark) Human Cytokine Assay (Bio-Rad Laboratories). It should be noted that the measurement kit used in the Example 1 is a kit which measures the concentrations without distinguishing between the active IL-18 and the IL-18 precursor. The average value of the serum IL-18 concentration in healthy controls was 142.2 pg/mL, whereas that in the PM/DM-ILD patients was 319.4 pg/mL, showing a statistically significant elevation in the PM/DM-ILD patients (analysis by an unpaired t-test, p < 0.0001). Accordingly, an antibody that recognizes and neutralizes the active IL-18 is can be effective for treating the PM/DM-ILD patients.

### [Example 2] Concentration of Active IL-18 in Serum of PM/DM-ILD Patients

Next, the concentrations of the active IL-18 in the serum of the patients were evaluated using the antibody that recognizes the active IL-18. The serum was collected from patients diagnosed with PM/DM between February 2007 and February 2023 at Nagasaki University Hospital, at the first medical examination. For serum samples from healthy individuals, those collected at routine checkups were selected as age- and sex-matched controls. The cases in which the ILD was detected by the HRCT were classified as the PM/DM-ILD. The disease type was classified as follows: cases showing progression of symptoms or radiographic infiltrates within one month after the onset were defined as acute; cases showing the progression of the symptoms or the radiographic infiltrates within one to three months were defined as subacute; and other cases were defined as chronic. The measurement was conducted in the same manner as in the Example 1, except that the Human Active IL-18 ELISA Kit (mAbProtein Co., Ltd.) was used in order to detect the active IL-18. Comparison of the active IL-18 levels between the two groups was performed using the Mann-Whitney U test, with p < 0.05 considered as statistically significant.

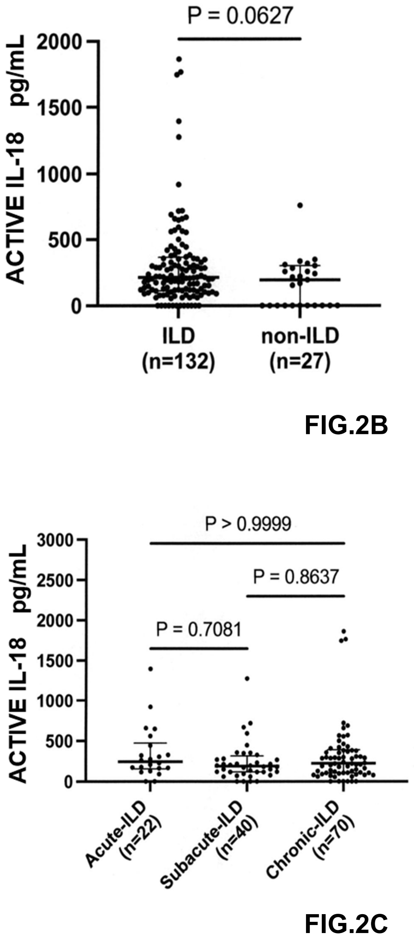

The serum concentrations of the active IL-18 of the PM/DM-ILD patients and healthy controls were measured. The concentration of the active IL-18 in the PM/DM-ILD patients (n = 132) (the median: 213 pg/mL; the interquartile range: 113 to 366) was significantly higher than the concentration of the active IL-18 in the healthy controls (n = 34) (the median: 0 pg/mL; the interquartile range: 0 to 204) (Figure 2A, p < 0.0001). Among the PM/DM-ILD patients, comparison of the concentrations of the active IL-18 between the cases complicated by the ILD and the cases not complicated by the ILD was performed. It showed that the concentrations of the active IL-18 in the cases not complicated by the ILD (n = 27) (the median: 196 pg/mL; the interquartile range: 0 to 303) was not statistically significantly different from that in the cases complicated by the ILD, but the concentration tended to be higher in the cases complicated by the ILD (Figure 2B, p = 0.0627). The cases were classified by the disease type, and the concentrations of the active IL-18 were examined. In the three groups, that is, acute cases (n = 22; the median: 245 pg/mL; the interquartile range: 155 to 474); subacute cases (n = 40; the median: 189 pg/mL, the interquartile range: 118 to 316), and chronic cases (n = 70; the median: 225 pg/mL; the interquartile range: 105 to 391), no statistically significant differences were observed in the concentrations of the active IL-18 in the serum (Figure 2C).

### [Example 3] Concentrations of Active IL-18 in Bronchoalveolar Lavage Fluid

Bronchoalveolar lavage fluid collected at the first medical examination from patients diagnosed with the PM/DM-ILD at Nagasaki University Hospital between March 2007 and January 2022 were measured for the concentrations of the active IL-18. For samples from healthy individuals, bronchoalveolar lavage fluid samples which had been stored at the Nagasaki University Hospital and could be collected were used. As with the Example 2, the disease type was classified as follows: cases showing progression of the symptoms or the radiographic infiltrates within one month after the onset were defined as acute; cases showing the progression of the symptoms or the radiographic infiltrates within one to three months were defined as subacute; and other cases were defined as chronic. The concentrations of the active IL-18 in the bronchoalveolar lavage fluid were measured using the Human Active IL-18 ELISA Kit. The IL-18 levels between the two groups were compared using the Mann-Whitney U test.

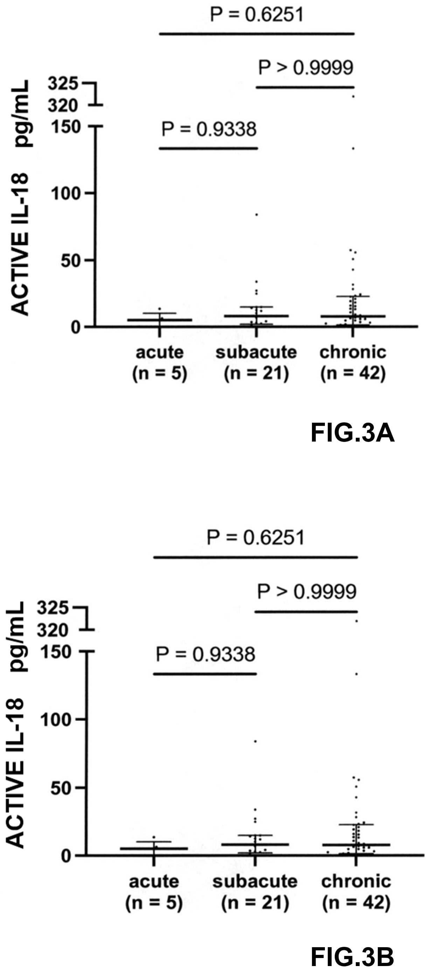

As in the Example 2, the concentrations of the active IL-18 in the bronchoalveolar lavage fluid were compared between the patients and the healthy individuals. The concentration of the active IL-18 in the bronchoalveolar lavage fluid of the PM/DM-ILD patients (n = 68) (the median: 7.0 pg/mL; the interquartile range: 1.4 to 19.0) was significantly higher than the concentration of the active IL-18 in the healthy controls (n = 12) (the median: 0 pg/mL; the interquartile range: 0 to 2.8) (Figure 3A, p < 0.0008). Meanwhile, when the cases were classified by the disease type, among the three groups, that is, acute cases (n = 5; the median: 5.3 pg/mL; the interquartile range: 0 to 10.1); subacute cases (n = 21; the median: 8.1 pg/mL, the interquartile range: 2.0 to 14.8), and chronic cases (n = 42; the median: 7.8 pg/mL; the interquartile range: 1.3 to 22.8), no statistically significant differences were observed in the concentrations of the active IL-18 in the bronchoalveolar lavage fluid (Figure 3B).

For samples in which both the serum and the bronchoalveolar lavage fluid were obtained from the same patient, the correlation between the active IL-18 concentrations in the serum and the bronchoalveolar lavage fluid was analyzed using a Pearson's correlation coefficient (Figure 3C). A positive correlation was observed between the concentration of the active IL-18 in the serum and the concentration of the active IL-18 in the bronchoalveolar lavage fluid (r = 0.45, p = 0.0003).

These results demonstrate that the concentration of the IL-18 and the active IL-18 in the serum of the PM/DM-ILD patients are significantly higher than the concentrations in the serum of the healthy individuals. Furthermore, since the concentrations of the active IL-18 in the bronchoalveolar lavage fluid were also significantly elevated as compared with those in the healthy controls, it is suggested that a treatment of PM/DM-ILD with an anti-active IL-18 antibody could be effective. Moreover, as shown in Figure 2C and Figure 3B, the concentrations of the active IL-18 in both the serum and the bronchoalveolar lavage fluid were high regardless of the disease type (acute, subacute, or chronic), indicating that an antibody against the active IL-18 may be effective irrespective of the disease type.

### [Example 4] Analysis (1) Using a Mouse Pulmonary Fibrosis Model - Comparison with IL-18BP

The rodents model with pulmonary fibrosis induced by bleomycin has long been used as an animal model for screening antifibrotic drugs. As described above, the IL-18BP has been reported to exhibit an effect in a mouse with pulmonary fibrosis induced by bleomycin. Accordingly, the effect of an anti-IL-18 antibody recognizing a neo-epitope was evaluated using such mice with pulmonary fibrosis induced by bleomycin. Since the neo-epitope differs between mouse and human IL-18, an antibody recognizing the mouse neo-epitope was used to verify the effect of the anti-IL-18 antibody.

A polypeptide including residues 37 to 193 generated by caspase cleavage from human inflammatory cytokine IL-18 protein (Uniprot Q14116; SEQ ID NO: 15) functions as active IL-18. Although the mouse IL-18 (NP_001344150; SEQ ID NO: 16) and the human IL-18 share high sequence homology, their N-terminal sequences that form the neo-epitope of the active IL-18 are different (Figure 4A; the C-terminal side of the arrow in Figure 4A functions as the active IL-18, and the cleaved fragment forms the neo-epitope). A mouse IL-18 neo-epitope sequence (sequence NFGRLHCTTC; SEQ ID NO: 17) was synthesized by a standard method. This is a peptide corresponding to the residues 36 to 44 of the mouse IL-18, which form the cleaved fragment upon the cleavage by caspase, with an additional cysteine (C) at the C-terminus. It was crosslinked to the peptide of SEQ ID NO: 17 using keyhole-limpet hemocyanin (hereinafter, KLH) using the Imject Maleimide-Activated mcKLH Spin Kit (Thermo Scientific). Thereafter, mice were immunized with the peptide by a standard method, and hybridomas were screened and established (Patent Literature 6).

Among the obtained hybridomas, the 5-4.1 antibody produced by the 5-4.1 hybridoma was used for the following experiments. The isotype of the 5-4.1 monoclonal antibody was confirmed to be IgG1, κ, using the IsoStrip Mouse Monoclonal Antibody Isotyping Kit (Sigma). It was confirmed that the 5-4.1 antibody recognized and neutralized only the mouse active IL-18 by ELISA, immunoprecipitation, and IL-18 functional inhibition assays. Only the results of the alanine scanning method with ELISA are shown (Figure 4B). As described above, when a protein is cleaved by a protease, such as caspase, a neo-epitope, that is, a protein cleaved fragment not normally present in that protein, is formed. Epitope analysis of the neo-epitope peptide used for the antibody production was performed by the alanine scanning method. Here, ELISA was used for a quantitative analysis.

Peptides corresponding to the N-terminal sequence peptide (residues 36 to 44, NFGRLHCTT; SEQ ID NO: 18) of the mouse active IL-18, and peptides with biocytin added to the C-terminus of alanine mutants of the N-terminal four amino acids (SEQ ID NOS: 19 to 22) were synthesized by a standard method. Each biocytin-added peptide was immobilized on a NeuroAvidin plate via biotin, and the recognition site of the 5-4.1 antibody was analyzed by ELISA (Figure 4B). Absorbance values obtained with a plate reader are shown to the right of the ELISA image, relative to the value for the wild-type peptide as 100, and presented with standard deviation.

The 5-4.1 antibody exhibited very low recognition of only 4% and 6% toward the peptide (N36A, SEQ ID NO: 19) in which the first amino acid of the N-terminal sequence peptide of the mouse active IL-18 protein (asparagine (N) at residue 36) was changed to alanine (A), and the F37A peptide (SEQ ID NO: 20) mutant peptides, and also showed weak reactivity of only 13% for the G38A (SEQ ID NO: 21) mutant. Further, the reactivity to the R39A (SEQ ID NO: 22) mutant was 116%, slightly stronger than that of the wild type. The results of the alanine scanning method revealed that the peptide from residues 36 to 38 is particularly important for recognition by the 5-4.1 antibody. That is, the 5-4.1 antibody was confirmed to be an antibody that recognizes NFGRLHCTT (SEQ ID NO: 18) as an epitope and NFG as a minimum epitope, and to be an antibody that recognizes a neo-epitope of the mouse IL-18.

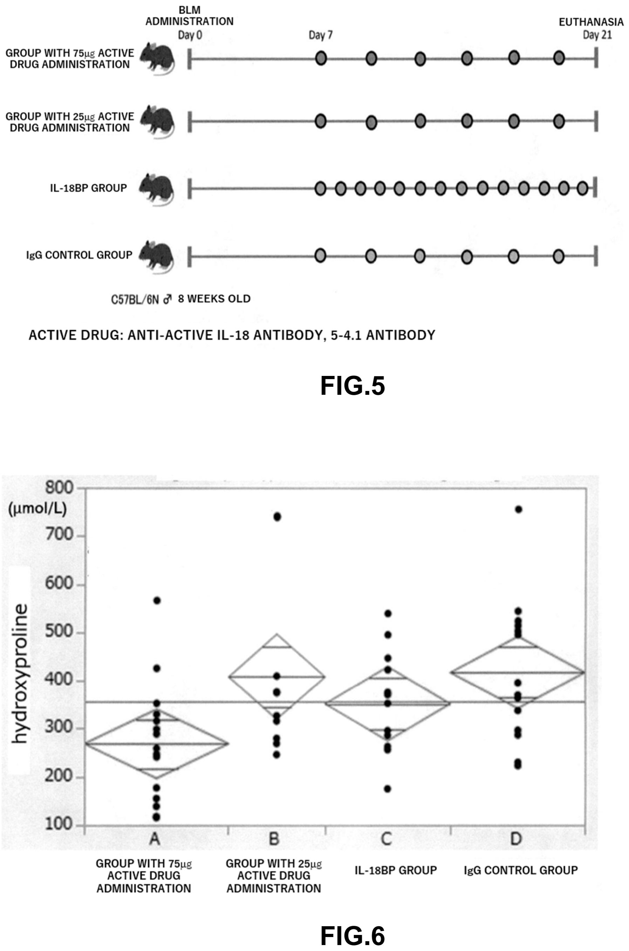

Next, the effect of the anti-active IL-18 antibody on the interstitial lung disease model was analyzed. The experimental schedule is shown in Figure 5. Male C57BL/6NJcl mice of eight weeks old (Charles River Japan) were anesthetized via intraperitoneal injection of a mixture of three types of anesthetic agents (medetomidine hydrochloride 7.5 µg/100 µL, midazolam 40 µg/100 µL, and butorphanol tartrate 50 µg/100 µL) in an amount of 0.1 mL per 10 g body weight, at the start of the experiment (day 0 of the experiment), and then received a single intratracheal administration of bleomycin (indicated as BLM in Figure 5; Nippon Kayaku Co., Ltd.) in an amount of 1 mg/kg to create a pulmonary fibrosis mouse model. Starting one week later, the anti-active IL-18 antibody (the 5-4.1 antibody; mAbProtein Co., Ltd.) was administered intraperitoneally in an amount of 200 µL/body, at a concentration of 25 µg/200 µL or 75 µg/200 µL, three times per week for two weeks (in total, six doses; the groups are referred to as the "group with 25 µg active drug administration " and the "group with 75 µg active drug administration," respectively). Control groups similarly received intraperitoneal administration, but of an IgG antibody (Mouse IgG1 κ isotype control; Bio X Cell) in an amount of 75 µg/200 µL per body (in total, six doses; the group is referred to as the "IgG control group"), or IL-18BP (R&D) in an amount of 5 µg/200 µL per body daily for 14 days (in total, 14 doses; the group is referred to as the "IL-18BP group") (each group included 15 mice, and the group with 25 µg active drug administration included 10 mice.). On the day 21 after the administration of the bleomycin, mice were euthanized by CO₂ asphyxiation, then autopsied, and both lungs were harvested. A homogenate was prepared from the right lung for measurement of hydroxyproline content in the lung tissue, which is a biochemical marker of pulmonary fibrosis (analysis outsourced to SRL Inc.), and a histopathological sample of the left lung was prepared.

The results of the measurement of the hydroxyproline content, which is the biochemical marker of the pulmonary fibrosis, are shown (Figure 6). In the group with 75 µg active drug (the 5-4.1 antibody) administration, the hydroxyproline content was significantly reduced as compared with the IgG control group to which the IgG was administered (p = 0.029). Also in the group with mouse IL-18BP administration, a decreasing trend was observed as compared with the control group, but the difference was not statistically significant as compared with the control group. In other words, it can be regarded that the anti-active IL-18 antibody exerts a superior therapeutic effect on the IL-18BP.

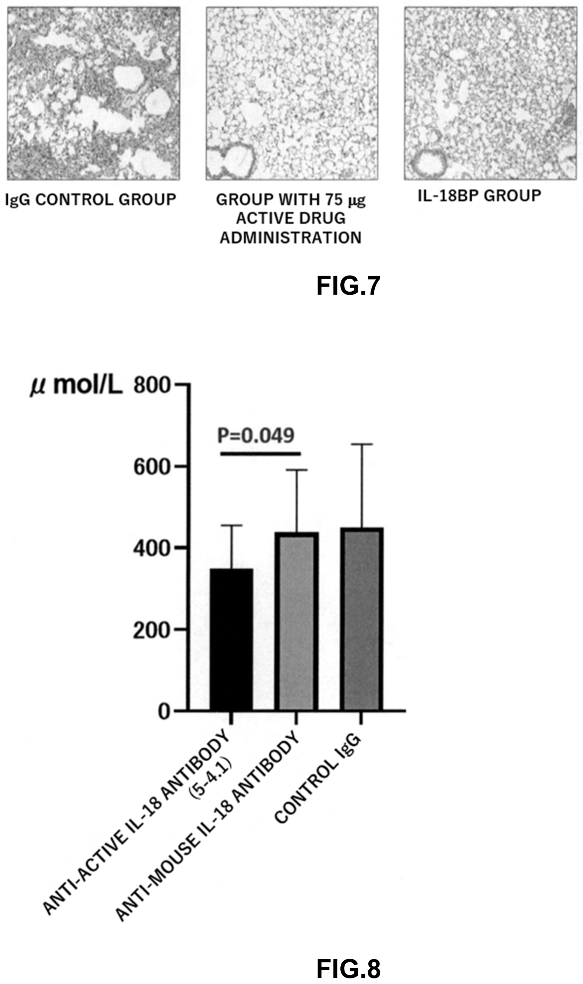

In addition, histopathological observations of lung tissue from each group were made using hematoxylin-eosin (HE) stained samples. Representative tissue images from each group are shown (Figure 7). Inflammation with destruction of the pulmonary alveoli induced by the bleomycin was clearly suppressed in the group with 75 µg active drug (the 5-4.1 antibody). In contrast, while the IL-18BP group showed a tendency toward suppression as compared to the IgG control group, the destruction of the pulmonary alveoli and inflammatory changes were observed more than in the group with 75 µg active drug administration.

### [Example 5] Analysis (2) Using a Mouse Pulmonary Fibrosis Model - Comparison with Anti-IL-18 Antibody

Using the same experimental schedule as in the Example 4, the mouse model with the pulmonary fibrosis induced by the bleomycin was treated with the 5-4.1 antibody recognizing the active IL-18 and an antibody recognizing the full length of the IL-18 (Bio X Cell, Cat. No. BE0237), each administered in an amount of 75 µg per mouse, and their effects were compared. The hydroxyproline contents in the lung tissue are shown (Figure 8). In the mice administered with the anti-active IL-18 antibody (the 5-4.1 antibody), the hydroxyproline contents were significantly lower than in the group administered with the anti-IL-18 antibody.

As demonstrated above, this study is the first to show, using an animal model, that an anti-active IL-18 antibody exerts a therapeutic effect on an interstitial lung disease. As indicated in the above Examples, anti-IL-18 antibodies that recognize the IL-18BP or regions other than the neo-epitope exhibited little or no effect in the mouse pulmonary fibrosis model, but the anti-active IL-18 antibody was observed to have a significant therapeutic effect. Accordingly, the anti-active IL-18 antibody can be used for the treatment of interstitial lung diseases for which no effective therapeutic drug has previously been available.

## Claims

1. A pharmaceutical composition for treating an interstitial lung disease, including as an active ingredient an anti-active IL-18 neutralizing antibody, or a fragment thereof selected from Fab, Fab', F(ab')₂, single chain antibody (scFv), disulfide stabilized V region fragment (dsFv), antibody with a reduced molecular weight (Fv-clasp), or a peptide including a CDR.

2. The pharmaceutical composition for treating an interstitial lung disease according to claim 1, wherein the interstitial lung disease is an interstitial lung disease associated with polymyositis and/or dermatomyositis.

3. The pharmaceutical composition for treating an interstitial lung disease according to claim 1 or 2, wherein the anti-active IL-18 neutralizing antibody includes a heavy chain variable domain or an antigen binding site that binds to an N-terminal region of active IL-18, including a CDR1H region consisting of the amino acid sequence GFSLSSSGMG (SEQ ID NO: 1), a CDR2H region consisting of the amino acid sequence IWWDDDK (SEQ ID NO: 2), and a CDR3H region consisting of the amino acid sequence TRTRTYSNFGGGMAY (SEQ ID NO: 3), and a light chain variable domain of the anti-active IL-18 neutralizing antibody is a peptide including a CDR1L region consisting of the amino acid sequence QSIAHSNGYTY (SEQ ID NO: 4), a CDR2L region consisting of the amino acid sequence KVS, and a CDR3L region consisting of the amino acid sequence VQGSHVPLT (SEQ ID NO: 5); or wherein the heavy chain variable domain of the anti-active IL-18 neutralizing antibody includes a CDR1H region consisting of the amino acid sequence GFSLTSYG (SEQ ID NO: 6), a CDR2H region consisting of the amino acid sequence IWAGGST (SEQ ID NO: 7), and a CDR3H region consisting of the amino acid sequence ARESSYDAMDY (SEQ ID NO: 8), and the light chain variable domain of the anti-active IL-18 neutralizing antibody is a peptide including a CDR1L region consisting of the amino acid sequence ENVVTY (SEQ ID NO: 9), a CDR2L region consisting of the amino acid sequence GAS, and a CDR3L region consisting of the amino acid sequence GQGYSYPYT (SEQ ID NO: 10).

4. The pharmaceutical composition for treating an interstitial lung disease according to claim 1 or 2, wherein the amino acid sequence of the H chain variable region of the anti-active IL-18 neutralizing antibody or the antigen binding site binding to the N-terminal region of the active IL-18 is SEQ ID NO: 11, and the amino acid sequence of the L chain variable region is SEQ ID NO: 12; or wherein the amino acid sequence of the H chain variable region is SEQ ID NO: 13 and the amino acid sequence of the L chain variable region is SEQ ID NO: 14.

5. A therapeutic effect prediction method for a patient with an interstitial lung disease as a subject to determine in advance whether the pharmaceutical composition for treating the interstitial lung disease according to any one of claims 1 to 4, the method including measuring an expression level of IL-18 in the subject.
